# EUROPEAN PATENT APPLICATION

(11) **EP 2 070 493 A1**
(43) Date of publication of application: **17.06.2009**
(21) Application number: 07800840.6
(22) Date of filing: 31.08.2007
(51) Int. Cl.: A61F 2/82, A61M 36/04, A61N 5/10

(54) **DUCT STENT BEING CAPABLE OF CARRYING THE SUBMINIATURE RADIOACTIVE PARTICLE SOURCE**

(30) Priority: 21.09.2006 CN 200610116321
(71) Applicant: The Second Military Medical University, Shanghai 200433 (CN)
(72) Inventor: LU, Zheng, Shanghai 200433 (CN); LIU, Yan, Shanghai 200433 (CN); LI, Zhao-Shen, Shanghai 200433 (CN)
(74) Representative: Schweiger, Martin
(86) International application number: PCT/CN2007/002623
(87) International publication number: WO 2008/034341

(57) **Abstract**

A duct stent being capable of carrying the subminiature radioactive particle source is mainly used for the inside radiation therapy of metaphase or terminal pancreatic cancer and bile duct cancer, and has drainage function. The duct stent consists of a tubular drainage tube (1) and fixation portions (2) which are face to face with two ends of the drainage tube (1) and can fix the stent to prevent the stent from moving. The lumen of the drainage tube (1) is a drainage cavity (1.1) for draining the pancreatic juice or the bile. A particle passage (1.2) is provided in the tube wall of the drainage tube (1). The inside diameter of the particle passage (1.2) matches with the outer diameter of the subminiature radioactive particle source.

## Description

### FIELD OF THE INVENTION

The present invention relates to a duct stent for carrying miniature radioactive particle sources in the field of medical devices, which is applied to internal radiation therapy of metaphase/advanced pancreatic cancer or cholangiocarcinoma and provides a drainage function.

### BACKGROUND OF THE INVENTION

Pancreatic cancer and cholangiocarcinoma is a group of serious tumors in digestive tracts with high lethal risk, low cure rate and poor prognosis. Most of these cancers are often diagnosed in the advanced period thereof, and it is too late to carry out the surgical resection. The tumors usually oppress pancreatic duct and common bile duct, and result in stenosis and occlusion. The clinical symptoms of stenosis or obstruction are mainly alleviated by the placement of the duct stent having the drainage function through endoscopy. The duct stents are mainly divided into plastic stents and metal stents.

The common plastic duct stent includes a drainage tube and barbs formed on the outer surface of two opposite ends of the drainage tube in clinics. The duct stent is placed into the affected part through duodenoscopy, so that the drainage tube supports on an obstruction or narrow region. The bile or the pancreatic juice can be drained through a drainage cavity in the drainage tube. The barbs on the two opposite ends can be used to position the duct stent, so as to avoid the duct stent from falling into the digestive cavity, and also to prevent the duct stent from completely falling into the pancreatic duct or the common bile duct. The duct stent can be removed and replaced regularly in order to maintain effective drainage.

The metal stent is widely used to treat the benign and malignant stenosis in the body lumen of human, and has a metal mesh structure which can support and fix the affected part by the function of self-expansion way. Some researchers process the metal stent by chemically electroplating to evenly plate radionuclides on the metal stent, so as to finish metal stents with different radioactive intensity for being applied to prevent the lumen restenosis generated by endothelial proliferation of blood vessels or bile ducts after implanting an internal stent. Other researchers attach a polyurethane film with radionuclides of ¹⁶⁶Ho to the surface of the metal stent for treating the esophageal cancer or the lumen restenosis generated after implanting an internal stent in the bile ducts. However, although the radioactive stents fabricated by the foregoing methods are used to treat diseases of malignant tumors, it can not satisfy the requirement of clinical therapy. There are some disadvantages, as follows: the manufacture process is complicated, the storage is inconvenient, and the dosage is single and can not be designed according to the tumor size of patients, so that the industrialization is difficult.

Presently, the miniature radioactive particle sources are applied to the local radiotherapy of pancreatic cancer and cholangiocarcinoma in the current clinical practice, and the curative effect thereof is demonstrated to be effective. Medical miniature radioactive particle are solid radioactive sources of short-rod shape, which are manufactured by packaging radionuclides in a titanium shell. Currently, the common particles are iodine (¹²⁵I) particles, palladium (¹⁰³Pd) particles, and so on. The length of the clinical specifications is 4.5mm and the diameter is 0.8mm. The radioactive particle sources can be placed into a part adjacent to the tumor or into the tumor through an implantation device for irradiation. The features include high local dosage of treated target part, low dosage of peripheral normal tissue, safe, reliable, easy to protection, and etc. The common implantation means is percutaneous implantation by an injection needle (or an implantation device having the injection needle) or implantation by a surgical operation. However, there are disadvantages including complicated operation, large traumatic wound, low recovery efficiency, and etc. After implantation, it can not be removed again, and can not adjust the therapy solution according to the curative effect.

### SUMMARY OF THE INVENTION

An object of the present invention is to provide a duct stent for carrying miniature radioactive particle sources, which can be placed into an affected part in pancreatic duct or bile duct through medical endoscopy. Thus, the convenience of placement operation can be increased, while the wound is miniaturized. Furthermore, the duct stent can provide a drainage function and used to partially irradiate peripheral tumors. Meanwhile, the duct stent can be removed and replaced regularly in accordance with requirements. The manufacture process of the duct stent is simple, and the duct stent can be designed according to the position and the volume of tumors. The local radiation dosage of the duct stent can be adjusted according to the optimal dose calculation in aid of a computer, in order to achieve the best therapeutic effect.

In order to solve the above problem, the present invention provides a duct stent for carrying miniature radioactive particle sources, comprising: a drainage tube having a tubular structure; and fixation portions formed on two opposite ends of the drainage tube for positioning the duct stent to prevent the duct stent from shifting, wherein a lumen of the drainage tube is a drainage cavity for draining pancreatic juice or bile, characterized in that: a tube wall of the drainage tube is provided with a particle passage therein, and the particle passage has an inner diameter matched with an outer diameter of miniature radioactive particle sources placed in the particle passage.

The present invention further provides a duct stent for carrying miniature radioactive particle sources, comprising: a drainage tube and fixation portions formed on two opposite ends of the drainage tube for positioning the duct stent to prevent the duct stent from shifting, wherein a lumen of the drainage tube is a drainage cavity for the draining pancreatic juice or bile cavity, characterized in that: a tube wall of the drainage tube is provided with a particle groove thereon, the particle groove has a size matched with an outer diameter of miniature radioactive particle sources placed in the particle groove, and the particle groove can be selectively sealed according therapy needs after placing the radioactive particle sources.

The above-mentioned drainage tube having the tubular structure is made of plastic material.

The above-mentioned drainage tube has a cross-section of arbitrary regular shape or irregular shape.

The above-mentioned fixation portions formed on two opposite ends of the drainage tube are barbs opposite to each other for positioning the duct stent to prevent the duct stent from shifting.

The above-mentioned particle passage provided in the tube wall of the drainage tube is parallel to the drainage tube, while the particle passage has a length equal to that of the drainage tube. The inner diameter of the particle passage is matched with the outer diameter of the placed miniature radioactive particle sources, and radionuclide sources are embedded in the tube wall of the drainage tube by placing different types of radionuclide sources in the particle passage of the duct stent.

During placing the radioactive particles, the above-mentioned particle passage provided in the tube wall of the drainage tube is selectively processed by a certain material to seal a space portion between two or a plurality of the adjacent miniature radioactive particle sources in the particle passage according to therapy needs.

The outer diameter of the above-mentioned duct stent is selectively varied according to the diameter of bile duct and pancreatic duct, while a plurality of the particle passages are provided according to the distribution of cancer portions for placing and fixing various types of miniature radionuclide sources.

An outer portion of the tube wall of the above-mentioned drainage tube for placing the miniature radioactive particle sources is formed with irradiation windows, wherein the size, the shape and the pitch of the irradiation windows are dependent upon the shape or the intensity of a radiation source, the size of the irradiation windows is matched with the miniature radioactive particle sources by controlling the length and the width of the irradiation windows slightly less than the size of the miniature radioactive particle sources, so as to conveniently observe the position of the radioactive particle sources in the tube wall and prevent the placed miniature radioactive particle sources from departing from the irradiation windows. Thus, the radionuclides can be safely fixed on the tube wall of the drainage tube, while it can prevent the tube wall from shielding the radionuclides for carrying out the best therapeutic effect.

After placing radioactive particles, the above-mentioned irradiation windows are selectively processed by a certain material to cover or fill the irradiation windows according to therapy needs.

The duct stent can be designed into different specifications. For example, the outer diameter of the duct stent is varied according to the diameter of bile duct and pancreatic duct, while one, two, or a plurality of the particle passages are provided according to the distribution of cancer portions for placing and fixing various types of miniature radionuclide sources. According to therapy needs, one, two, or a plurality of the irradiation windows can be designed, while the size, the shape and the pitch of the irradiation windows are dependent upon the shape or the intensity of a radiation source. According to the shape of the miniature radionuclides, the miniature radionuclides can be directly placed and fixed in the irradiation windows or the particle groove of the tube wall without providing the particle passage. In use, the duct stent of corresponding specification is selected. The miniature radioactive particle sources are firstly placed in design positions of the duct stent, and then the duct stent is placed into a corresponding location in bile duct and pancreatic duct through duodenoscopy by conventional practices.

The cost of the present invention is low, the structure thereof is simple, and the convenience thereof is high. The present invention can expand bile duct and pancreatic duct to provide a drainage function, and used to place the miniature radioactive particle sources adjacent to peripheral tumors under the miniaturized wound for keeping efficiently irradiating the tumors for radiotherapy within a short distance. If necessary, the duct stent can be suitably removed and replaced, so as to be useful for patients to be treated and recover.

The present invention can also be placed into other parts of body by an endoscopy or other pathways for the radiotherapy of tumors.

### DESCRIPTION OF THE DRAWINGS

Figure 1 is a schematic diagram of the overall structure of the present invention;
Figure 2 is a longitudinal cross-section diagram of the present invention;
Figure 3 is a schematic diagram of Embodiment 3;
Figure 4 is a schematic diagram of Embodiment 4; and

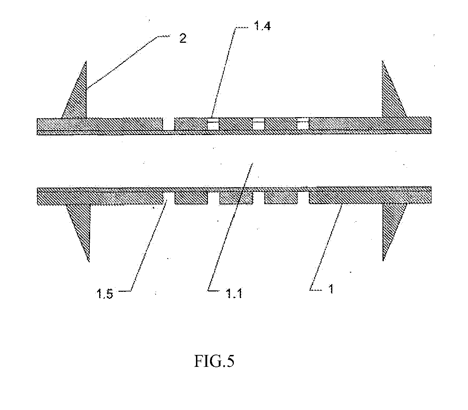

Figure 5 is a schematic diagram of Embodiment 5.

### DETAILED DESCRIPTION OF THE PREFERRED EMBODIMENTS

The present invention is described more detailed by drawings and embodiments.

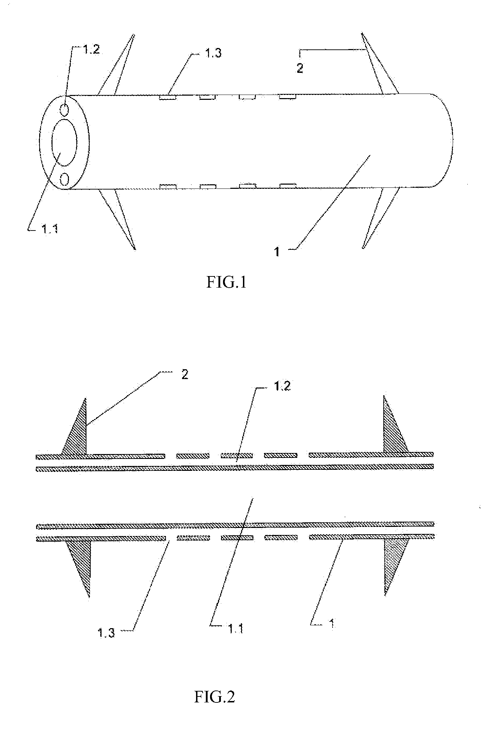

The duct stent of the present invention comprises a drainage tube 1 and opposite barbs 2 formed on two opposite ends of the drainage tube 1, as shown in Figs. 1 and 2. An internal lumen of the drainage tube 1 is a drainage cavity 1.1. A tube wall of the duct stent is provided with a particle passage 1.2 therein, the particle passage 1.2 has an inner diameter matched with an outer diameter of miniature radioactive particle sources. An outer side wall of the particle passage 1.2 is formed with irradiation windows 1.3. The length and the width of the irradiation windows 1.3 are slightly less than the miniature radioactive particle sources.

In use, according to the therapy solution, appropriate miniature radioactive particle sources and corresponding duct stent are selected, and then the miniature particles are inserted into the particle passage 1.2 of the duct stent in turn, so as to fixe the miniature particles in the irradiation windows 1.3.

Then, the duct stent is placed into common bile duct or pancreatic duct through an endoscope by conventional practices. The location of the duct stent is adjusted under the X-ray fluoroscopy, so that the miniature radioactive particle sources are located near the lesion region. The barbs 2 can help to position the duct stent.

### Embodiment 1: A pancreatic duct stent for carrying miniature radioactive particle sources

The length of the duct stent is 54.4 mm, and the outer diameter thereof is 3.2 mm. The diameter of the drainage cavity is 1.8 mm. One of the particle passage is provided in the tube wall, and the inner diameter thereof is 0.8 mm. Three of the irradiation windows are formed on the outer side wall of the particle passage, wherein the length, the width and the height thereof are 4.4x0.7x0.2 mm, respectively. The pitch between centers of adjacent irradiation windows is 10.0 mm. The distance from each of the two outermost irradiation windows to each of the two ends of the duct stent is 15 mm. The length of each of the two barbs is 7 mm, wherein the length from the root of the barbs to the distal end of the duct stent is 5 mm. The length of the miniature radioactive particle sources is 4.5 mm, and the diameter thereof is 0.8 mm. The activity of a single particle is 0.5 mCi. The pancreatic duct stent which is designed to have a treatment dosage of 50 Gy for supporting the radioactive particle sources ¹²⁵I is successfully placed into a pig pancreatic duct through the endoscopy, so that the duct stent is demonstrated to be safe and effective.

### Embodiment 2: A bile duct stent for carrying miniature radioactive particle sources

The length of the duct stent is 69.4 mm, and the outer diameter thereof is 3.6mm. The diameter of the drainage cavity is 1.5 mm. Two of the particle passages are provided in the tube wall and opposite to each other, and the inner diameter thereof is 0.8 mm. Six of the irradiation windows are formed on the outer side wall of each of the particle passages, wherein the length, the width and the height are 4.4x0.7x0.2 mm, respectively. The pitch between centers of adjacent irradiation windows is 5.0 mm. The distance from the frontmost window to the front end of the duct stent is 25 mm, while the distance from the rearmost window to the rear end of the duct stent is 15 mm. The length of each of the two barbs is 7 mm, wherein the length from the root of the barbs to the distal end of the duct stent is 5 mm. The length of the miniature radioactive particle sources is 4.5 mm, and the diameter thereof is 0.8 mm. The activity of a single particle is 0.6 mCi. The bile duct stent which is designed to have a treatment dosage of 150 Gy for supporting the radioactive particle sources ¹²⁵I is successfully placed into a pig bile duct through the endoscopy, so that the duct stent is demonstrated to be safe and effective.

According to tumor conditions of patients and difference of irradiation purposes, it can select if the irradiation window is provided based on the requirement by using different material of the duct stent, i.e. different shielding functions of the irradiation of the radioactive sources. Embodiments 3 and 4 are described more detailed, as follows:

### Embodiment 3:

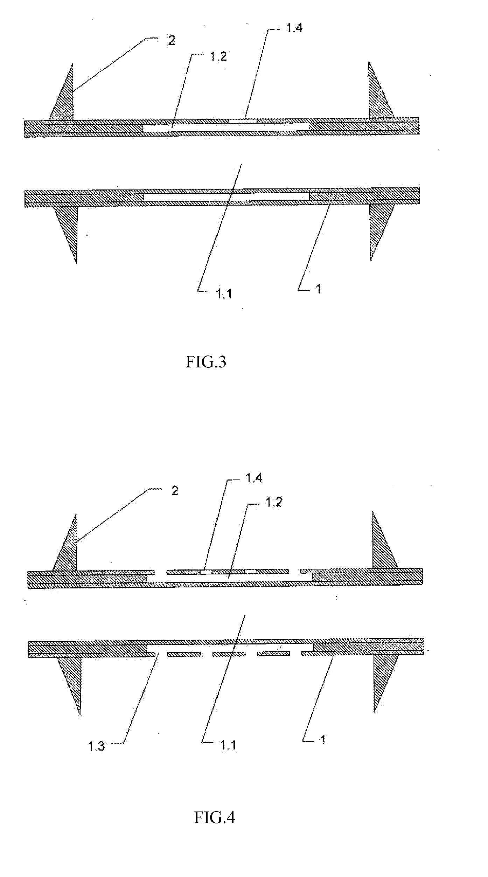

As shown in Fig. 3, the duct stent is made of low-shielding material, such as polyethylene without providing the irradiation window 1.3. A wide irradiation range can be carried out, while the irradiation effect is not affected, so as to be suitably applied to various tumors. Alternatively, after providing the irradiation window 1.3, the low-shielding material 1.4 is used to cover or fill the irradiation window, in order to prevent the radioactive sources from falling out.

### Embodiment 4:

As shown in Fig. 4, the duct stent is made of high-shielding material or irradiation-impenetrable material, and provided with the irradiation window 1.3, so as to be suitably applied to local irradiation of tumors to prevent from irradiating other normal tissues unnecessary to be irradiated. Alternatively, after providing the irradiation window 1.3, the low-shielding material 1.4 is used to cover or fill the irradiation window, in order to prevent the radioactive sources from falling out.

### Embodiment 5:

The tube wall of the duct stent can be provided with the particle groove 1.5 which has a size matched with the outer diameter of the placed miniature radioactive particle sources, wherein the radioactive sources are fixed in the groove for the purpose of positioning the radioactive sources on the tube wall. Alternatively, after providing the particle groove 1.5, the low-shielding material 1.4 is used to cover or fill the particle groove 1.5, in order to prevent the radioactive sources from falling out.

The present invention has been described with a preferred embodiment thereof and it is understood that many changes and modifications to the described embodiment can be carried out without departing from the scope and the spirit of the invention that is intended to be limited only by the appended claims.

## Claims

1. A duct stent for carrying miniature radioactive particle sources, comprising: a drainage tube (1) having a tubular structure; and fixation portions (2) formed on two opposite ends of the drainage tube for positioning the duct stent to prevent the duct stent from shifting, wherein a lumen of the drainage tube (1) is a drainage cavity (1.1) for draining pancreatic juice or bile, **characterized in that**: a tube wall of the drainage tube (1) is provided with a particle passage (1.2) therein, and the particle passage has an inner diameter matched with an outer diameter of miniature radioactive particle sources placed in the particle passage.

2. A duct stent for carrying miniature radioactive particle sources, comprising: a drainage tube (1) and fixation portions (2) formed on two opposite ends of the drainage tube for positioning the duct stent to prevent the duct stent from shifting, wherein a lumen of the drainage tube (1) is a drainage cavity (1.1) for the draining pancreatic juice or bile cavity, **characterized in that**: a tube wall of the drainage tube (1) is provided with a particle groove (1.4) thereon, and the particle groove has a size matched with an outer diameter of miniature radioactive particle sources placed in the particle groove.

3. The duct stent according to claim 1 or 2, **characterized in that**: the drainage tube (1) having tubular structure is made of plastic material.

4. The duct stent according to claim 1 or 2, **characterized in that:** the drainage tube (1) has a cross-section of arbitrary regular shape or irregular shape.

5. The duct stent according to claim 1 or 2, **characterized in that**: an outer portion of the tube wall of the drainage tube (1) for placing miniature radioactive particle sources is formed with irradiation windows, wherein the size, the shape and the pitch of the irradiation windows are dependent upon the shape or the intensity of a radiation source, the size of the irradiation windows is matched with the miniature radioactive particle sources by controlling the length and the width of the irradiation windows slightly less than the size of the miniature radioactive particle sources, so as to prevent the placed miniature radioactive particle sources from departing from the irradiation windows.

6. The duct stent according to claim 1 or 2, **characterized in that**: the fixation portions (2) are barbs.

7. The duct stent according to claim 1, **characterized in that**: the particle passage (1.2) provided in the tube wall of the drainage tube (1) is parallel to the drainage tube (1).

8. The duct stent according to claim 1, **characterized in that**: the particle passage (1.2) provided in the tube wall of the drainage tube (1) has a length equal to that of the drainage tube (1).

9. The duct stent according to claim 1, **characterized in that**: the inner diameter of the particle passage (1.2) is matched with the outer diameter of the placed miniature radioactive particle sources by placing different types of radionuclide sources in the particle passage (1.2) to embed the radionuclide sources in the tube wall of the drainage tube (1).

10. The duct stent according to claim 1, **characterized in that**: a space portion between two or a plurality of the adjacent miniature radioactive particle sources in the particle passage (1.2) is sealed according to therapy needs.

11. The duct stent according to claim 1, **characterized in that**: the outer diameter of the particle passage (1.2) is varied according to the diameter of bile duct and pancreatic duct, while a plurality of the particle passages are provided according to the distribution of cancer portions for placing and fixing various types of miniature radionuclide sources.
